Europäisches Patentamt

European Patent Office

Office européen des brevets

(19)

(11) Veröffentlichungsnummer : **0 098 234**
**B1**

(12) **EUROPÄISCHE PATENTSCHRIFT**

(45) Veröffentlichungstag der Patentschrift :
30.08.89

(21) Anmeldenummer : 83730056.5

(22) Anmeldetag : 21.06.83

(51) Int. Cl.⁴ : **A 01 G 7/00**

(54) Verfahren zur Herstellung von planzlichem Vermehrungsmaterial.

(30) Priorität : 28.06.82 HU 208582

(43) Veröffentlichungstag der Anmeldung :
11.01.84 Patentblatt 84/02

(45) Bekanntmachung des Hinweises auf die Patenterteilung : 30.08.89 Patentblatt 89/35

(84) Benannte Vertragsstaaten :
AT BE CH DE FR GB IT LI NL

(56) Entgegenhaltungen :
WO-A-81/032 55
US-A- 3 683 550
US-A- 3 972 146
PHYSIOLOGIA PLANTARUM, vol. 54, 1982, Kopenhagen L. JOHANSSON et al. :"Improvement of anther
culture technique: Activated charcoal bound in agar
medium in combination with liqiud medium and elevated CO2 concentration" (received 12 May 1981) Seiten 24-30

(73) Patentinhaber : NOVOTRADE RT.
Fürst Sándor u. 24-26
H-1136 Budapest (HU)

(72) Erfinder : Molnár, György, Dr. Dipl.-Ing.
Vöröshadsereg u. 200
H-1039 Budapest (HU)
Erfinder : Tétényi, Péter, Dr. Dipl.-Ing.
Népstadion u. 9
H-1134 Budapest (HU)
Erfinder : Dobos, Eva, Dipl.-Ing.
Vérmezö u. 10-12
H-1012 Budapest (HU)
Erfinder : Bernáth, Jenö, Dr. Dipl.-Ing.
Nyár u. 93
H-1045 Budapest (HU)

(74) Vertreter : Maikowski, Michael, Dipl.-Ing. Dr.
Xantener Strasse 10
D-1000 Berlin 15 (DE)

EP 0 098 234 B1

## Beschreibung

Die Erfindung betrifft ein Verfahren zur Herstellung von pflanzlichem Vermehrungsmaterial durch vegetative Mikrovermehrung in sterilen Gewebekulturen.

Es ist bekannt, daß insbesondere bei zweijährigen und mehrjährigen Pflanzen, Sträuchern, Schmuck- und Zimmerpflanzen die vegetative Vermehrung zahlreiche Vorteile hat. Aus dem bewurzelten Trieb wächst die komplette Pflanze schneller als aus dem Samenkorn ; auch ist die vegetative Vermehrung einfacher. Ihr Hauptvorteil ist jedoch, daß die von einem bestimmten Exemplar auf vegetativem Wege gewonnenen Nachkommen genetisch als ein einziges Individuum anzusehen sind, d.h. ihre Eigenschaften sind — von durch verschiedene Umwelteinflüsse hervorgerufenen unwesentlichen Abweichungen abgesehen — identisch. Dies ist von außerordentlicher Bedeutung in Fällen, in denen ein infolge seiner Eigenschaften wertvolles Exemplar zur Verfügung steht (zum Beispiel eine Arzneipflanze mit hohem Alkaloidgehalt, ein besonders frostbeständiger, ertragreicher Beerenstrauch), durch dessen Vermehrung ein homogener Bestand mit ausgezeichneten Eigenschaften gewonnen werden kann.

Eine der herkömmlichen gärtnerischen Methoden der vegetativen Vermehrung besteht darin, den Spitzentrieb oder einen anderen Trieb der zu vermehrenden Pflanze abzuschneiden und — gegebenenfalls nach Bewurzeln in Wasser — in einen Blumentopf, im Gewächshaus oder unter freiem Himmel in die Erde zu pflanzen. Bei manchen Pflanzen können auch durch Teilen der Wurzel oder der Wurzelknollen vegetative Nachkommen erzielt werden, jedoch ist in allen diesen Fällen die Zahl der von einem Exemplar anziehbaren Nachkommen recht beschränkt.

Deswegen werden besonders wertvolle Pflanzen (zum Beispiel Arzneipflanzen) in steriler Gewebekultur, zum Beispiel Organ-, vor allem jedoch Triebkultur, gezüchtet. Bei diesem auch als Mikrovermehrung bezeichneten Verfahren wird aus dem keimfrei gemachten pflanzlichen Trieb in an sich bekannter Weise eine Triebkultur angelegt. Die auf diese. Weise gewonnenen Triebe werden in mit Agar-agar verfestigten Nährboden gesteckt, wo sie weiter wachsen und sich verzweigen. Der feste Nährboden kann gegebenenfalls die für die jeweilige Pflanzenart experimentell bestimmte optimale Kombination pflanzlicher Hormone enthalten. Die Methoden der pflanzlichen Zell-, Gewebe- und Organkultur werden z. B. in der folgenden Literatur beschrieben : Applied and Fundamental Aspects of Plant cell, Tissue and Organ Culture ; Redakteure : Reinert und Bajaj : Springer-Verlag Berlin-Heidelberg-New York, 1977.

Die erhaltenen Triebe und Triebanlagen werden abgetrennt, bewurzelt und ggf. nach einer Übergangszeit im Gewächshaus ausgepflanzt. Mit diesem Verfahren konnte, wenn auch nicht für alle Pflanzen, die Anzahl der von einer Pflanze erhältlichen Setzlinge wesentlich erhöht werden.

Nach einem weiterentwickelten Verfahren ist es bekannt, die Gewebekultur in einem festen Nährboden anzuziehen und die Triebkultur anschließend in eine Nährlösung zu überführen, die einen Wachstumsregulator enthält. Bei diesem bekannten Verfahren können die Pflanzenstecklinge Nährstoffe nur aus der Nährlösung durch Diffusion aufnehmen (Staba et al. WO-A-81/03255).

Der Erfindung liegt die Aufgabe zu Grunde, die bekannten Verfahren so weiter zu entwickeln, daß die Wirtschaftlichkeit der Mikrovermehrung in sterilen Gewebekulturen erheblich gesteigert bzw. für bestimmte Pflanzen die Mikrovermehrung überhaupt erst in wirtschaftlich vertretbarer Weise anwendbar wird.

Die Erfindung geht von der Überlegung aus, daß das Pflanzenwachstum besser und kräftiger wird, wenn die Pflanze die Nährstoffen nicht nur an ihrem unteren Pol bzw. durch die dort bereits ausgebildete Wurzel, sondern mit ihrer gesamten Oberfläche aufnimmt. Zwar ist der feste Nährboden für die Induzirung des Wurzelwachstums geeignet, da er unmittelbar mit der Schnittfläche des Pflanzenteils in Berührung kommt, jedoch sind die Stoffe, die die Ausbildung von Trieben bzw. allgemein deren Entwicklung stimulieren von der Stelle ihrer Wirkungsentfaltung ziemlich weit entfernt. Die Wirkung der Nährstoffe kann gesteigert werden, wenn sie nicht durch die Wurzel aufgenommen werden, sondern unmittelbar mit dem Trieb in Berührung kommen.

Um diese beiden Gedanken zu realisieren, hätte es nahe gelegen, ähnlich der in der Landwirtschaft verbreitet angewendeten Laubdüngung die in Wasser gelösten Nährstoffe (z. B. Mikroelemente) durch Versprühen auf die Oberfläche der Pflanzen auszubringen. Unter den Bedingungen der sterilen Technik läßt sich die Sprühmethode jedoch nicht anwenden. Außerdem wäre die erzielte Wirkung der Nährstoffe nur vorübergehend.

Überraschenderweise wurde nun gefunden, daß die Pflanzen nicht nur nicht geschädigt werden, sondern stärker wachsen, sich besser verzweigen und mehr Triebe bilden, wenn man die in festem Nährboden wachsende Triebkultur völlig mit Nährlösung auffüllt, d. h. vorübergehend in eine submerse Kultur umwandelt. Das ist sehr überraschen, da sich die normalerweise über der Erde befindlichen Teile der Landpflanzen daran angepaßt haben, von Luft umgeben zu assimilieren und zu atmen und es daher nicht zu erwarten war, daß die vegetative Vermehrbarkeit der in der Art von Aquariumspflanzen kultivierten Triebe um eine Größenordnung ansteigt.

Gegenstand der Erfindung ist demnach ein Verfahren zur Herstellung von pflanzlichem Vermehrungsmaterial durch Mikrovermehrung in sterilen Gewebekulturen unter Verwendung von Nährlösung. Das erfindungsgemäße Verfahren besteht aus den im Anspruch 1 beschriebenen Schritten.

Zur Verfestigung des festen Nährbodens verwendet man zweckmäßig Agar-agar. Der feste Nährboden gewährleistet die Grundversorgung der Pflanze mit Nährstoffen, die durch die Gefäßbündel weitergeleitet werden. Weiterhin ist der feste Nährboden erforderlich, damit die Triebe eine den natürlichen Bedigungen entsprechende, d. h. senkrechte Lage einnehmen können. Die flüssige Nährlösung versorgt die Triebkultur mit zusätzlichen Nährstoffen. Die von der Mineralsalze, Zucker und Vitamine enthaltenden hormonfreien Nährlösung überfluteten Kulturen bilden Triebe, die 30-100 % dicker und länger sind als die der nicht submers wachsenden Kontrollkulturen. Der flüssigen Nährlösung können jedoch auch noch pflanzliche Hormone zugesetzt werden. Von diesen seien die Auxine, Cytokine und Gebberelline erwähnt. Von den Cytokinen hat sich besonders das 6-Benzyl-aminopurin (6-Benzylandenin) als vorteilhaft erwiesen. Durch die Wirkung der Pflanzenwachstumsregulatoren kommt es in der submersen Kultur zu einer massenhaften Entwicklung von Trieben. Diese Entwicklung ist etwa und 100-500 % intensiver als bei einer Kontrollkultur, bei der der Wachstumsregulator in den Nährboden gegeben wurde. In der mit cytokinhaltiger Nährlösung überfluteten Kultur erfolgt die Bildung von Triebknospen und Triebspitzen an der gesamten Oberfläche des submersen Pflanzenteils ; sogar die neugebildeten Triebe verzweigen sich weiter.

Der in der Flüssigkeit befindliche Teil der submersen Kultur sieht etwa aus wie ein Weihnachtsbaum oder wie ein Bündel wild wuchernde Wasserpflanzen und hat nicht die geringste Ähnlichkeit mit nomalen Exemplaren seiner Art. Die Triebspitzen, die aus der Flüssigkeit herausgewachsen sind, bilden nur wenig oder gar keine Seitentriebe. Diese Triebspitzen werden abgeschnitten und in an sich bekannter Weise bewurzelt. Die aus ihnen angezogenen Pflanzen werden völlig normal.

Der unter Flüssigkeit stehende Teil der Kultur, der nach dem Abschneiden der Spitzentriebe übrigbleibt, weist zahlreiche Knospen und Seitentriebe auf. Dieses Rumpfstück wird unter sterilen Bedingungen in Stücke geschnitten. Die dabei erhaltenen Stecklinge werden auf die bereits beschriebene Weise in festen Nährboden gesteckt, mit flüssiger Nährlösung aufgefüllt, und das Verfahren wird so lange wiederholt, bis die gewünschte Anzahl auspflanzbarer Triebe erreicht ist.

Wahrscheinlich deshalb, weil die Bedingungen für Assimilation und Atmung in der Flüssigkeit anders sind als in der Luft (Lichtabsorption, die Gase liegen in Wasser gelöst vor usw.), hat es sich als vorteilhaft erwiesen, wenn die submerse Kultur länger beleuchtet wird als üblich beziehungsweise, wenn sie ununterbrochen beleuchtet wird.

Die Erfindung wird mit Hilfe der folgenden Beispiele näher erläutert, ist jedoch nicht auf diese Beispiele beschränkt.


## Beispiel 1

Submerse Vermehrung von Amsonia tabernaemontana in Gegenwart eines Wachstumsreglers

Aus dem Amsonia tabernaemontana Bestand des Ungarischen Forschungsinstitutes für Arzneipflanzen wurde unter den 10-15 Jahre alten, auf ihren Wirkstoffgehalt untersuchten Pflanzen ein Exemplar mit 12,26 %. Tabersonin-Gehalt ausgesucht. Im April wurden von dieser Pflanze 25-30 cm lange Triebe mit etwa 20 Blättern abgeschnitten. Die Triebe wurden dann — der besseren Handhabbarkeit halber — in etwa 10 cm lange Stücke geschnitten und eine Stunde lang unter fließendem Leitungswasser gewaschen. Dann wurden die Blätter auf etwa ein Viertel ihrer Größe zurückgeschnitten. Die so zugestutzten Triebstücke wurden in 3 %iger Natriumhypochloritlösung unter ständigem Schütteln 15 Minuten lang keimfrei gemacht. Aus der Hypochloritlösung wurden die Pflanzenteile in im Autoklav sterilisiertes destilliertes Wasser überführt und unter Schütteln 10 Minuten lang gewaschen. Die letztere Maßnahme erfolgte ebenso wie die folgenden unter aseptischen Bedingungen in einem sterilen Impfschrank. Die dem sterilen Wasser entnommenen Triebe wurden in einer durch Wärme sterilisierten Petrischale mit einem Skalpell in je 2-3 Blätter tragende Stücke geschnitten, wobei unter dem untersten Blatt ein etwa 1 cm langes Triebstück belassen wurde. Die auf diese Weise vorbereiteten Stecklinge wurden in Glaskolben des Volumens 200 ml gesteckt, die je 100 ml modifizierten Murashige-Skoog-Nährboden enthielten. Dieser hatte folgende Zusammensetzung :

| | | |
|---|---:|---|
| $NH_4NO_3$ | 1 650 | mg/l |
| $KNO_3$ | 1 900 | mg/l |
| $CaCl_2 \cdot 2H_2O$ | 440 | mg/l |
| $MgSO_4 \cdot 7H_2O$ | 370 | mg/l |
| $KH_2PO_4$ | 170 | mg/l |
| $Na_2EDTA$ | 37,2 | mg/l |
| $FeSO_4 \cdot 7H_2O$ | 27,8 | mg/l |
| $H_3BO_3$ | 6,2 | mg/l |
| $MnSO_4 \cdot 7H_2O$ | 22,3 | mg/l |
| $ZnSO_4 \cdot 7H_2O$ | 8,6 | mg/l |
| $KJ$ | 0,83 | mg/l |
| $Na_2MoO_4 \cdot 2H_2O$ | 0,25 | mg/l |
| $CuSO_4 \cdot 5H_2O$ | 0,025 | mg/l |
| $CoCl_2 \cdot 6H_2O$ | 0,025 | mg/l |
| Saccharose | 30 000 | mg/l |

| Agar-agar | 10 000 | mg/l |
|---|---|---|
| Nicotinsäure | 0,5 | mg/l |
| Pyridoxin · HCl | 0,5 | mg/l |
| Thiamin · HCl | 0,1 | mg/l |

(Murashige, T., Skoog, F. : A revised medium for rapid growth and bioassays with tobacco tissue cultures, Physiol. Plantarum 15, 473-497/1962/). Beim Einsetzen wurde darauf geachtet, daß die Stecklinge bis zum ersten Blatt im Nährboden stecken. Die Kolben wurden bei 20-25 °C gehalten, während der täglich 16 Stunden künstlicher Beleuchtung betrug die Beleuchtungsstärke 10 000 lux. Nach einer Woche erschienen in den Blattachseln Triebknospen. Aus diesen wuchsen in den folgenden zwei Wochen etwa 5-7 cm lange, 10-12 Blätter aufweisende Triebe.

Diese sterilen Seitentriebe wurden abgetrennt und in Stücke mit je 2-4 Blättern geschnitten. Diese Setzlinge wurden wenigstens 1 cm tief in den Nährboden der obigen Zusammensetzung gesteckt (deshalb muß beim Zuschneiden ein entsprechend langes Stück unter dem letzten Blatt gelassen werden).

Nun wurde das gleiche Nährmedium noch einmal angesetzt, jedoch ohne Agar-agar, statt dessen mit 1 mg/l 6-Benzylaminopurin. Von dieser flüssigen Nährlösung wurden in jeden der 200-ml-Kolben 50 ml gegossen. Die unter der Nährlösung stehenden Stecklinge wurden dann bei 20-25 °C ständig mit 8000 lux beleuchtet.

In sämtlichen Blattachseln bildeten sich Triebknospen, aus denen innerhalb von 3-4 Wochen 5-10 cm lange Triebe wuchsen. Für diese war charakteristisch, daß sie auch ihrerseits wieder Knospen und Triebe bildeten. Die stärksten Triebe wuchsen aus dem flüssigen Nährmedium heraus ; ihr in der Luft befindlicher Teil bildete in den Blattachseln wenig oder keine Triebknospen mehr. Nun wurde die Kultur aufgeteilt. Die 5-7 Blätter tragenden Triebspitzen wurden abgeschnitten, in den festen Nährboden der angegebenen Zusammensetzung gesteckt und nach 2 Wochen, als sich schon reichlich Wurzeln gebildet hatten, in Erde gepflanzt.

Das verbliebene Unter- und Mittelstück der Kultur wurde in Stücke mit je 2-4 Blättern bzw. 5-10 Knospen geschnitten. Diese wurden ebenfalls in den festen Nährboden der obigen Zusammensetzung gesteckt und dann — auf die bereits beschriebene Weise — mit der flüssigen Nährlösung übergossen, die 1 mg/l 6-Benzylaminopurin enthielt. Die Kulturen wurden bei 20-25 °C und ständiger Beleuchtung von 8000 lux Beleuchtungsstärke 2-4 Wochen lang kultiviert. Während dieser Zeit wachsen die bereits vorhandenen Seitentriebe, verzweigen sich weiter und bilden neue Knospen. Die entsprechend entwickelten Spitzentriebe werden abgeschnitten und bewurzelt, der Rest der Kultur wird wieder in Knospen und Triebstücke geschnitten.

Durch systematische Wiederholung des Verfahrens können aus einem einzigen Trieb im Laufe eines Jahres mehrere Millionen bewurzelte Pflanzen hergestellt werden.

## Beispiel 2

Submerse Vermehrung von Estragon (Artemisia dracunculus) ohne Wachstumsregulator

Von im Freiland gezogenen Estragonpflanzen (Artemisia dracunculus), Sorte Grüne Würze, wurden im Juni etwa 10 cm lange Triebe abgeschnitten. Die Triebe wurden zwei Stunden lang in fließenden Leitungswasser gewaschen, dann in 3 %iger Natriumhypochloritlösung 10 Minuten lang sterilisiert, hinterher mit sterilem destilliertem Wasser 20 Minuten gespült und dann unter sterilen Bedingungen in etwa 3 cm lange, 2-4 Blätter aufweisende Stücke geschnitten. Diese wurden in den Nährboden gemäß Beispiel 1 gesteckt. Die Kolben mit den Stecklingen wurden mit der flüssigen Nährlösung gemäß Beispiel 1 aufgefüllt. Die Nährlösung enthielt jedoch keinen Pflanzenwachstumsregulator.

Durch die Wirkung der flüssigen Nährlösung beginnen die Triebkulturen intensiv zu wachsen. Das Wachstum ist 2-5 mal so stark wie das der Kontrolle ohne flüssiges Nährmedium. Die Kulturen werden bei 20-25 °C unter ständiger künstlicher Beleuchtung von 8 000 lux Beleuchtungsstärke gehalten. Die in den 100 ml Nährboden enthaltenden Kolben von 200 ml Volumen befindlichen Triebkulturen hatten in 4-5 Wochen den Luftraum der Kolben völlig zugewachsen. Die 8-15 cm langen Triebe waren 2-3 mm dick, turgeszent und verhältnismäßig wenig verzweigt.

Die Triebkulturen wurden in 2-4 Blätter aufweisende Stücke geschnitten, die sowohl zum Anwurzeln wie auch zur Züchtung weiteren Triebmaterials geeignet waren.

Zur Bewurzelung werden die Triebstecklinge am besten für 24-96 Stunden in den Nährboden gemäß Beispiel 1 gesteckt, der zusätzlich noch 1 mg/l 2,4-Dichlorphenoxyessigsäure enthält. Dann werden die Stecklinge in Nährboden ohne Wachstumsregler überführt, wo sich in 2 Wochen an jedem Steckling 5-10 jeweils 2-5 cm lange Wurzeln bilden. Die bewurzelten Pflanzen werden, wenn Material zum Auspflanzen gewonnen werden soll, ohne flüssige-Nährlösung gehalten.

Man kann jedoch die bewurzelten Pflanzen auch unter flüssige Nährlösung setzen. In diesem Falle tritt gleichzeitig massenweise Triebvermehrung ein. Durch systematische Wiederholung des Verfahrens können in einem Jahr aus einem Triebstück 100 000 bewurzelte Pflanzen hergestellt werden.

## Beispiel 3

4

Catharanthus roseus (Vinca rosea), submerse Vermehrung in Gegenwart von Kinetin

Von den im Gewächshaus stehenden Catharanthus-Pflanzen wurden 10-15 cm lange Seitentriebe geschnitten. Diese wurden 4 Stunden lang unter fließendem Leitungswasser gewaschen, dann in 1 %iger Natriumhypochloritlösung 25 Minuten lang liegen gelassen und anschließend in sterilem destilliertem Wasser 2×15 Minuten gespült. Die auf 2-4 Blätter zurückgeschnittenen Stecklinge wurden auf die im Beispiel 1 beschriebene Weise in festen Nährboden gesteckt, bei 25-28 °C gehalten und täglich 16 Stunden mit einer Beleuchtungsstärke von 10 000 lux beleuchtet. Die Spitzenstecklinge wuchsen weiter, verzweigten sich jedoch im allgemeinen nicht; aus den aus Seitentrieben gewonnenen Stecklingen hingegen trieben meistens zwei Seitentriebe.

Nach 3-4 Wochen wurden die entstandenen Seitentriebe und die gewachsenen Spitzentriebe zerschnitten und die Stücke in den festen Nährboden gemäß Beispiel 1 gesteckt. Die Kolben mit den Stecklingen wurden mit je 50 ml flüssiger Nährlösung aufgefüllt. Die Zusammensetzung der Nährlösung entsprach dem in Beispiel 1 angegebenen Rezept mit dem Unterschied, daß die Lösung kein Agar-agar enthielt, dafür jedoch in einer Konzentration von 1 mg/l Kinetin. In den mit der Lösung aufgefüllten Kolben wurden die Seitentriebe 2-3 mal so dick wie die der auf festem Nährboden ohne Überfluten gezogenen Kontrolle.

Je öfter die erhaltenen Triebe weitergeimpft werden, um so intensiver wird die Triebentwicklung, und auch die Wurzelbildung der Spitzentriebe wird besser. Durch systematische Wiederholung des Verfahrens können aus einem einzigen Spitzentrieb in einem Jahr etwa 100 000 Spitzentriebe gewonnen werden.

## Patentansprüche

1. Verfahren zur Herstellung von pflanzlichem Vermehrungsmaterial durch Mikrovermehrung in sterilen Gewebekulturen unter Verwendung von Nährlösung, wobei man

1. sterile Stecklinge einer Pflanze in festen Nährbodensteckt,

2. die Stecklinge mit einer ggf. wachstumsregulierende Stoffe enthaltenen Nährlösung vollständig über flutet und die Triebe in diesem submersen System wachsenläßt, und

3. die entstandenen verzweigten, viele Seitenknospen und -triebe tragenden Kulturen in Stücke schneidet, die dabei anfallenden Spitzentriebe bewurzelt und nach einer Übergangszeit aus dem Gewächs haus ins Freilandpflanzt, die aus dem unteren und dem Mittelstück der sich vollständig in Flüssigkeit befindlichen Triebkultur stammenden Stecklinge in das unter 1 und 2 beschriebene Verfahren als Ausgangsmaterial zurückführt und die Schritte 1, 2 und 3 des Verfahrens bis zum Erreichen dergewünschten Anzahl von Spitzentrieben wiederholt.

2. Verfahren nach Anspruch 1, dadurch gekennzeichnet, daß der feste Nährboden mit Agar-agar verfestigt wird.

3. Verfahren nach Anspruch 1 und 2 dadurch gekennzeichnet, daß der flüssigen Nährlösung als wachstumsregulierende Substanz ein Cytokinin, vorzugsweise 6-Benzyl-aminopurin zusetzt wird.

4. Verfahren nach einem der Ansprüche 1 bis 3, dadurch gekennzeichnet, daß die submerse Kultur täglich für wenigstens 16 Stunden beleuchtet wird.

## Claims

1. Method for the production of plant propagation material by micro-propagation in sterile tissue cultures, using nutrient solution, in which

1. sterile sets of a plant are planted into solid nutrient soil,

2. the sets are completely inundated with a nutrient solution which may contain growth-regulating substances and the shoots are allowed to grow in this submerged system, and

3. the consequent branched cultures, which carry many side sprouts and shoots, are cut into pieces, the tip shoots then obtained are rooted and, after a transition time, planted out of the greenhouse into open ground, the sets deriving from the lower piece and middle piece of the shoot culture situated completely in liquid are returned as starting material into the method as described under 1 and 2 and the steps 1, 2 and 3 of the method are repeated until the desired number of tip shoots is reached.

2. Method according to Claim 1, characterised in that the solid nutrient soil is consolidated with agar-agar.

3. Method according to Claims 1 and 2, characterised in that a cytokinin, preferably 6-benzylaminopurine, is added as growth-regulating substance to the liquid nutrient solution.

4. Method according to one of Claims 1 to 3, characterised in that the submerged culture is illuminated for at least 16 hours daily.

## Revendications

1. Procédé de préparation de matériau de multiplication végétal par micromultiplication dans des cultures de tissus stériles en utilisant une solution nutritive (engrais liquide), dans lequel :

1. on place des boutures stériles d'une plante dans un milieu nutritif (engrais solide) ;

2. on submerge complètement les boutures avec un engrais liquide contenant éventuellement des matières régulant la croissance et l'on laisse pousser les pousses dans ce système submergé, et

3. on sectionne les cultures obtenues, ramifiées, comportant beaucoup de bourgeons latéraux et de pousses latérales en fragments, on munit de racines les bouts de pousses qui apparaissent et, au bout d'un temps de transition, on les transplante de la serre à l'air libre, on recycle dans le procédé décrit sous les N° 1 et 2, comme matériau de départ, les boutures provenant de la section inférieure et de la section centrale de la culture de pousses se trouvant complètement dans du liquide, et l'on répète les stades 1, 2 et 3 du procédé, jusqu'à l'obtention du nombre souhaité de bouts de pousses.

2. Procédé selon la revendication 1, caractérisé en ce qu'on consolide l'engrais solide avec de l'agar-agar.

3. Procédé selon les revendications 1 et 2, caractérisé en ce qu'on ajoute à l'engrais liquide, comme substance régulant la croissance, une cytokinine, de préférence la 6-benzylaminopurine.

4. Procédé selon l'une des revendications 1 à 3, caractérisé en ce qu'on éclaire la culture submergée tous les jours pendant au moins 16 heures.